(19) Europäisches Patentamt
European Patent Office
Office européen des brevets

(11) **EP 4 497 427 A1**

(12) **EUROPEAN PATENT APPLICATION**

(43) Date of publication:
**29.01.2025 Bulletin 2025/05**

(21) Application number: **23209859.0**

(22) Date of filing: **14.11.2023**

(51) International Patent Classification (IPC):
**A61F 13/42** (2006.01)  **A61F 13/514** (2006.01)

(52) Cooperative Patent Classification (CPC):
**A61F 13/42; A61F 13/51496;** A61F 2013/422;
A61F 2013/424; A61F 2013/8497

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.07.2023 EP 23187487**
**25.07.2023 EP 23187694**
**11.08.2023 EP 23191198**

(71) Applicants:
• **Ontex BV**
**9255 Buggenhout (BE)**

• **Ontex Group NV**
**9320 Erembodegem (BE)**

(72) Inventors:
• **HELLMOLD, Jens**
**59269 Beckum (DE)**
• **PEPERMANS, Manu**
**2018 Antwerpen (BE)**
• **MADAAN, Ankit**
**9000 Ghent (BE)**

(74) Representative: **Macchetta, Andrea**
**Ontex BV**
**Korte Keppestraat 21**
**9320 Erembodegem-Aalst (BE)**

(54) **ABSORBENT ARTICLE COMPRISING A SENSOR FOR EXUDATE DETECTION**

(57)    The present disclosure pertains to an absorbent article (7) comprising:

a. a liquid permeable topsheet (14)

b. a substantially liquid impermeable backsheet (16)

c. an absorbent core (15) positioned between said topsheet (14) and backsheet (16)

d. a sensor (6) for exudate detection, said sensor comprising an electrically conductive material.

According to the present disclosure, said absorbent article (7) further comprises indicia (30) positioned on at least one of a body-facing surface and garment-facing surface of the backsheet (16), and at least one of the following conditions is satisfied:

(i) said indicia (30) comprises an electrically conductive material and is arranged such that a plurality of islands (31) of said conductive material are formed on said backsheet (16); and

(ii) said indicia (30) has at least a first colour and the sensor (6) has at least a second colour and wherein a delta $E^*$ between the first colour and second colour is at least about 3, as measured according to the test method herein.

EP 4 497 427 A1

## Description

### TECHNICAL FIELD

The present disclosure is directed to monitoring systems and methods for absorbent articles comprising one or more sensors for automatic exudate detection. Generally the absorbent article being a disposable personal hygiene article selected from pants, diapers and pads for incontinence. The incontinence disposable personal hygiene articles such as pants, diapers and/or pads typically being for adults i.e. adult pants, adult diapers and/or adult pads.

### BACKGROUND

Absorbent articles for personal hygiene are designed to absorb and contain bodily exudates, such as a large quantity of urine. Non-limiting examples of disposable absorbent articles include diapers, pants, training pants, pads, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988.

A need however remains for systems and methods for improved error detection and correction related to exudate detection, particularly the location and/or amount thereof (most preferably location) within the absorbent article, and automatic product recognition and/or detection as well as easily indicating the absorbent article properties, such as size, absorbency, brand, product type so that a caregiver can easily and rapidly apply the appropriate article.

### SUMMARY

In a first aspect an absorbent article comprising:

    a. a liquid permeable topsheet;
    b. a substantially liquid impermeable backsheet;
    c. an absorbent core positioned between said topsheet and backsheet;
    d. a sensor for exudate detection, said sensor comprising an electrically conductive material;

**characterised in that** said absorbent article further comprises indicia positioned on at least one of a body-facing surface and garment-facing surface of the backsheet, and **in that** at least one of the following conditions is satisfied:

    (i) said indicia comprises an electrically conductive material and is arranged such that a plurality of islands of said conductive material are formed on said backsheet; and
    (ii) said indicia has at least a first colour and the sensor has at least a second colour and wherein a delta $E^*$ between the first colour and second colour is at least about 3, as measured according to the test method herein.

According to an embodiment, the delta $E^*$ is from about 3.5 to about 30, preferably from about 4 to about 25, even more preferably from about 4.5 to about 20, most preferably from about 5 to about 15, as measured according to the test method herein.

According to an embodiment, the indicia is selected from the group consisting of graphics, letters, numbers, patterns, icons, images, and combinations thereof.

According to an embodiment, the indicia comprises an electrically conductive material having one or more first conductive properties and the sensor comprises an electrically conductive material having one or more second conductive properties, and wherein the first and second conductive properties are different; preferably wherein the first conductive property comprises a first resistance and the second conductive property comprises a second resistance and wherein the first resistance is greater than the second resistance, preferably at least two times greater, more preferably between 2 and 5 times greater.

According to an embodiment, the indicia are positioned substantially between at least two electrodes of the sensor, preferably wherein at least two of said indicia are positioned between at least two same electrodes of the sensor.

According to an embodiment, the indicia is viewable from a garment facing surface of the absorbent article.

According to an embodiment, the indicia is used for both exudate detection and indication of one or more absorbent article properties, the one or more absorbent article properties preferably being selected from the group consisting of size, absorbency, brand, product type, and combinations thereof; more preferably wherein product type is selected from the group consisting of diapers, pants, pads, and combinations thereof.

According to an embodiment, the first colour has an $L_1$ of at least about 35 and the second colour has an $L_2$ of at most about 40, as measured according to the test method herein.

According to an embodiment, $L_1$ is from about 40 to about 80, preferably from about 45 to about 75, even more preferably from about 50 to about 70, as measured according to the test method herein.

According to an embodiment, $L_2$ is from about 0 to about 35, preferably from about 5 to about 30, even more preferably from about 10 to about 25, even more preferably from about 15 to about 20, as measured according to the test method herein.

According to an embodiment, the first colour has an $a_1$ of from about -40 to about 40, preferably from about -30 to about 30, as measured according to the test method herein.

According to an embodiment, the first colour has a $b_1$ of from about -80 to about 20, preferably from about -75 to about 10, even more preferably from about -70 to about 0, as measured according to the test method herein.

According to an embodiment, a portion of the sensor, preferably one or more terminal tracks of said sensor, are arranged to come into electrical contact with one or more conductive terminals of a reading device () preferably in the form of a clip-on device.

The present disclosure also pertains to an array comprising plurality of absorbent articles wherein said array comprises a first absorbent article, a second absorbent article, and preferably a third absorbent article, and wherein the indicia of each said article is different and that the first, second, and third absorbent articles are different in at least one of size, absorbency, product type, and brand.

According to an embodiment, the sensor for each said articles is different in at least one of shape, size, area-coverage, number of electrodes.

## BRIEF DESCRIPTION OF THE FIGURES

FIG. 1 Is an isometric view of a device according to an embodiment of the disclosure.

FIG. 2 Is an isometric view of a clip-on device according to an embodiment of the disclosure.

FIG. 3 is a plan view of an absorbent article according to an embodiment of the disclosure.

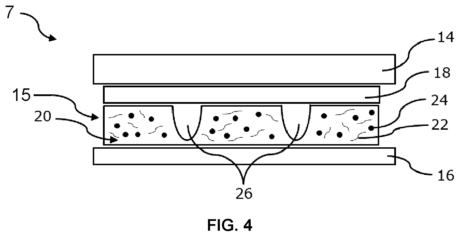

FIG. 4 illustrates a diagrammatic cross section of an absorbent article

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

As used herein the term "indicia" may comprise one or more inks with pigments, electrically conductive material, electrically conductive ink, adhesives with pigments, words, designs, trademarks, graphics, patterns, and/or pigmented areas, for example. Indicia is not merely a full colored or tinted layer, such as a backsheet layer, for example. The indicia may typically be a different colour than: (1) the layer that it is printed on, positioned on, or applied to; or (2) a different colour than other layers of an absorbent article. The phrase a "different colour" means a different shade of the same colour (e.g., dark blue and light blue) or may be completely different colour (e.g., blue and grey). The indicia should be at least partially visible from either a wearer facing surface, a garment facing surface, or both of an absorbent article, although the indicia may not be printed on, positioned or, on applied to the wearer or garment facing surfaces of the absorbent articles. The indicia may comprise an electrically conductive material, light activatable material, a liquid activatable material, a pH activatable material, a temperature activatable material, a menses activatable material, a urine activatable material, a BM activatable material. These activatable materials may typically undergo a chemical reaction, or other reaction, to change the indicia from one colour to a different colour, from one colour to a different shade of the same colour, from a colour that is not visually distinguishable or recognizable in an absorbent article to a colour that is visually distinguishable or recognizable in an absorbent article, or from a colour that is visually distinguishable or recognizable in an absorbent article to a colour that is not visually distinguishable or recognizable in an absorbent article. In an instance, the indicia may grow or shrink or display a graphic/not display a graphic after the indicia undergoes the reaction. In other instances, the indicia may be activated by a stress or a strain during manufacture or wear. The indicia may be white or non-white. If the indicia is white in colour, at least one layer may be non-white so that the indicia is visible from a wearer and/or garment facing surface of the absorbent articles, for example. The indicia may comprise embossments, fusion bonds, or other mechanical deformations. In other instances the indicia may at least partially overlap embossments, fusion bonds, or other mechanical deformations. In some instances, the indicia may be formed within either a sheath or a core of bicomponent fibers. For example, a core may be white, while a sheath may be blue, or vice versa. The indicia may be on, positioned on, formed on, formed with, printed on, or applied to all of, or part of, a certain layer. The indicia may also be on, positioned on, formed on, formed with, printed on, or applied to one or more layers of an absorbent article. The indicia may be on, positioned on, formed on, formed with, printed on, or applied to either side, or both sides, of the one or more layers of an absorbent article.

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined above and glue enclosed within the core wrap.

THE ARTICLE

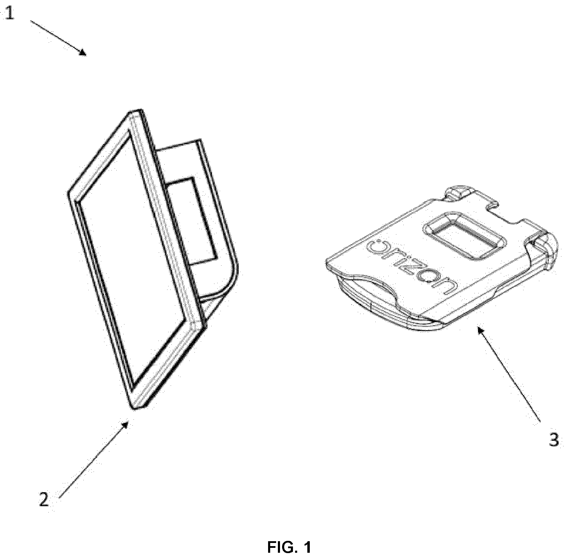

As exemplified in Fig. 1 to Fig.3, the absorbent article (7) herein is generally an incontinence article selected from the group consisting of a diaper, a pant, a pad, and combinations thereof. Preferably, incontinence disposable personal hygiene articles such as pants, diapers and/or pads being for adults i.e. adult pants, adult diapers and/or adult pads.

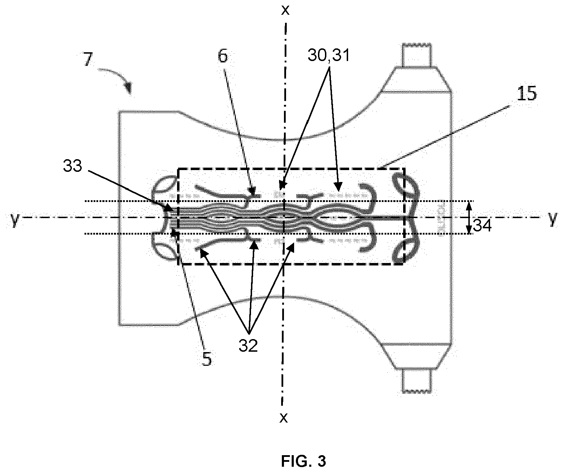

As exemplified in Fig. 3, the absorbent article (7) comprises a sensor (6) that may be applied onto a backsheet of said article. The article typically being constructed to comprise a liquid impermeable topsheet (14), a substantially liquid impermeable backsheet (16) and an absorbent core (15) positioned between the topsheet and backsheet. The sensor (6) may thus be located and/or positioned on a skin facing surface of the backsheet so that at least a portion of said sensor (6) is exposed to contact with exudates upon a voiding event. The sensor (6) generally being interposed directly and/or indirectly between the absorbent core and the backsheet. The distinct layers may be joined together by construction adhesive or the like means.

The sensor (6) comprises an electrically conductive material, preferably an electrically conductive ink, having a predetermined pattern. The pattern arranged to sense the presence of exudates in different locations of the absorbent article in an x-y cartesian coordinate system, typically by a change in one or more conductivity parameters such as change in resistance and/or capacitance and/or impedance.

The sensor (6) comprises a plurality of connection tracks (5) wherein more than two, preferably at least 3, even more preferably from 4 to 25, of the said plurality of connection tracks (5) may be in electrical communication with a sensing pattern (or sensing tracks) comprising a plurality of open electrical circuits disposed on a portion of the article (7), preferably the backsheet thereof, and arranged such that each said circuit is able to change one or more conductivity parameters when contacted with exudates and each said circuit being representative of one or more locations of a plurality of locations of an absorbent article in an x-y cartesian coordinate system. The plurality of connection tracks (5) are preferably at least 3, preferably at least 4, more preferably from 5 to 35, connection tracks (5). Examples of suitable sensor-comprising absorbent articles for testing herein are described in EP3760104.

The sensors for use herein may thus be used to determine the location of exudates within the absorbent article (e.g. by knowing which of the plurality of circuits are triggered) but not only, for example by measuring the signal strength (e.g. resistance value) an amount of exudate (e.g. in volume, mass or the like e.g. mL, g, etc.). Exudates herein may be selected from urine, stool, menses, and combinations thereof.

As illustrated in FIG. 3, the sensor (6), more particularly the connection tracks (5) can be partitioned in two portions, a central portion (34) and electrodes (32). The central portion (34) corresponds to the portion of the connection tracks (5) extending substantially longitudinally, meaning in the direction (y-y), preferably in the central area of the absorbent article (7) as illustrated in FIG.3, whereas the electrodes (32) correspond to the portion of the connection tracks (5) extending outboard of the central portion (34) with respect to the transversal direction (x-x) as illustrated in FIG. 3. In other words, the electrodes (32) extend transversely (along the x-x direction) beyond the central portion (34) of the connection tracks (5). In other words, the electrodes (32) are transversally, or laterally, outboard of the longitudinal centerline (y-y) of the absorbent article (7). In other terms, each electrode (32) comprises a proximal end and a distal end with respect to the central portion (32) or to the longitudinal centerline (y-y) and with respect to the transversal direction (x-x), said proximal end is connected to the central portion (34) and said distal end is the point furthest from the central portion (34) or from longitudinal centerline y-y. As illustrated in FIG. 3, the electrodes (32) can extend longitudinally and/or transversally. For sake of clarity, the connection tracks (5) comprise both central portion (34) and electrodes (32) or inversely, the central portion (34) and electrodes (32) are both parts or portions of the connection tracks (5), said electrodes (32) corresponding to the free-ends or extremities of the connection tracks (5) as illustrated in FIG. 3. The connection tracks (5) are in electrical communication with said electrodes 32 and central portion (34), meaning the connection tracks (5), central portion (34) and electrodes (32) are electrically connected. The electrodes (32) are arranged in different areas, or regions, of the absorbent article (7) such as in the front, sides, rear or the crotch area in order to have a proper location of the exudates. The electrodes (32) is the part of the connection tracks (5) that will more likely be in contact with exudates as they cover more different areas of the absorbent article (7).

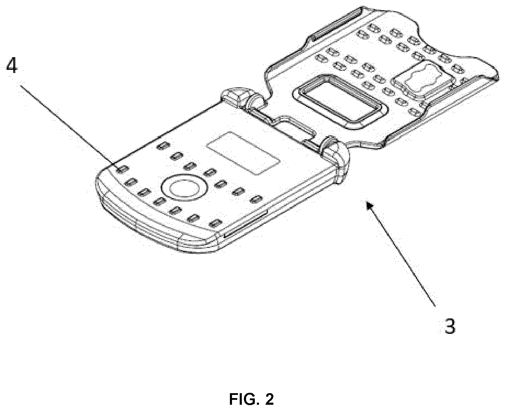

A clip-on device (3) may be connectable to the sensor (6) of the absorbent article (7). As exemplified in Fig. 2, the clip-on device (3) may comprise a power source, transmitter, a processing unit, and one or more electrical terminals (4) in electrical communication with at least one of the transmitter and the processing unit; said terminals (4) being adapted to come into contact with corresponding connection tracks (5) of a sensor (6). For example, as illustrated in FIG. 3, the terminals (4) can be adapted to come into contact with the terminal tracks (33) of the central portion (34) of the connection tracks (5), said terminal tracks being arranged at one longitudinal end of the backsheet (16). The clip-on device (3) may automatically acquire at least one of: exudate data comprising exudate information for one or more voiding events, preferably at least two different voiding events, within a time period corresponding to a monitoring period, the one or more voiding events corresponding to at least a first voiding event, and preferably a second voiding event, wherein the first, and preferably second, voiding event occurs at an absorbent article position [first and second voiding events can be different positions of the absorbent article]; and absorbent article data according to the one or more primary article characteristics that are selected from the group consisting of: absorbency of said article; size of said article; construction of said article; brand of said article; material specification of said article, and combinations thereof. Such data may be acquired directly to by the clip-on device (3) and transmitted directly or indirectly to a computing device (2). By indirectly it is intended herein that such data may be first transmitted to a further computing device comprising a logic and/or algorithm to process said data before being further transmitted to the computing device (2), the further computing device may be a cloud-based application.

As exemplified in Fig. 1, the computing device (2) may comprise a display, a processor and an input receiver and may be powered by a battery source or the like. The computing device (2) preferably being a hand-held device selected from the group consisting of a tablet, smartphone, portable computer, and combinations thereof.

The clip-on device (3) and the computing device (2) may thus be in direct or indirect data communication with each other. The data communication between the computing device (2) and the clip-on device (3) is preferably wireless. The wireless communication may comprise one or more of Bluetooth, Wi-fi, NFC, Zigbee, Z-Wave, and RFID. Preferably the wireless communication technology used employs radio waves and comprises frequency-hopping spread spectrum technique for secure and reliable communication. Preferably the computing device and the clip-on device are in Bluetooth wireless data communication.

The clip-on device (3) and computing device (2) may be generally referred to herein as data-capturing device or assembly (1).

FIG. 6 shows a diagrammatic cross section of a portion of the absorbent article to illustrate how the different components are arranged. The absorbent article (7) comprises a liquid permeable topsheet (14) that is arranged on the body-facing side of the absorbent article (7), a substantially liquid impermeable backsheet (16) that is arranged on the garment-facing side of the absorbent article, an absorbent core (15) positioned between said topsheet (14) and backsheet (16) and a sensor (6) (not shown in FIG. 6) for exudate detection, said sensor comprising an electrically conductive material. An optional acquisition distribution layer (18) can be positioned between said topsheet (14) and said absorbent core (15). The absorbent core (15) comprises absorbent material (20) for example selected from the group consisting of cellulose fibers (22), superabsorbent polymers (24) or a combination thereof. The absorbent core (15) can also optionally comprise at least one channel (26) substantially free of absorbent material. The topsheet (14) is associated to the backsheet (16). The topsheet (14) can be directly joined to backsheet 16 by affixing the topsheet (14) directly to the backsheet (16). Alternatively, the topsheet (14) can be indirectly joined to the backsheet (16 by affixing the topsheet (14) to intermediate members which in turn are affixed to the backsheet (16). Topsheet layer (14) and backsheet (16) can be affixed directly to each other or to intermediate members by attachment means such as an adhesive, sonic bonds, thermal bonds or any other attachment means known in the art. The absorbent article (7) can also comprise a topsheet (14) that has a portion directly joined to the backsheet (16 and another portion indirectly joined to the backsheet (16).

According to an embodiment, the backsheet (16) can be a unitary layer or a composite layer composed of multiple components assembled side-by-side or laminated . For example, the backsheet (16) may comprise a liquid impervious material in the form of a thin plastic film, e.g. a bio-polyethylene film, a nonwoven material coated with a liquid impervious material, a hydrophobic nonwoven material, which resists liquid penetration, or a laminate of a bioplastic film and a nonwoven material, the liquid impermeable material being on the body-facing side and the nonwoven material being on the garment facing side. The sensor (6) is preferably arranged on body facing side of the liquid impervious material in order to be closer to the exudates. According to a further embodiment, the backsheet (16) can further comprise an insulating strip arranged on the body-facing surface of backsheet (16) to sandwich a portion of the sensor (6) for example the connection tracks (5) so as to insulate the conductors from wetness or exudates. For example, the insulating strip can be arranged to cover only the central portion (34) of the connection tracks (5) thereby exposing the electrodes (32), *i.e.* the electrodes (32) are not covered by the insulating strip. The electrodes (32) and connection tracks (5) and central portion (34) being electrically connected, the system (e.g. absorbent article (7), clip-on (3) and computing device (2)) can still detect exudates whilst having a portion of the connectors insulated from exudates.

According to the present disclosure, the absorbent article (7) further comprises indicia (30) positioned on at least one of a body-facing surface and garment-facing surface of the backsheet (16). According to the present disclosure, at least one of the following conditions is satisfied:

(i) said indicia (30) comprises an electrically conductive material and is arranged such that a plurality of islands (31) of said conductive material are formed on said backsheet (16); and

(ii) said indicia (30) has at least a first colour and the sensor (6) has at least a second colour and wherein a delta $E^*$ between the first colour and second colour is at least about 3, as measured according to the test method herein.

In other words, the present disclosure pertains to an absorbent article (7) where one or both of the above conditions (i), (ii) is/are satisfied. Similarly, the indicia (30) can be positioned, or printed, on the body-facing surface of the backsheet (16) or on the garment-facing surface of the backsheet (16) or on both said surfaces.

Regarding condition (i), the term "a plurality of islands of said conductive material" as used herein means that the indicia (30) are arranged in portions that are isolated, or disconnected, from one another. Said indicia (30) can also be isolated from the connection tracks (5) such as the electrodes (32), the term "isolated" having its usual meaning such as separated, distinct, disconnected, apart, remote, not touching, *etc.* In other words, when considering the backsheet (16) alone and dry, *e.g.* before use, the indicia (30) are disconnected from each other and preferably disconnected from the sensor (6) e.g. from connection tracks (5) and electrodes (32). In other words, the electrically conductive material on the backsheet (16) is not arranged in a continuous manner or pattern. However, when a leakage or spill incident occurs whilst the absorbent article (7) is being worn, urine or other exudates, being composed of water and thus electrically conductive, the indicia (30) and the sensor (6) comprising connection tracks (5) and electrodes (32) become electrically connected via the urine or other exudates. More precisely, the indicia comprising electrically conductive material and therefore a resistance, will alter the current conducted by the exudates and affect conductivity, it is possible to detect this change in one or more conductivity parameters such as change in resistance and/or capacitance and/or impedance to further detect said exudates using the indicia (30) and electrodes (32) and central portion (34) which can all become electrically connected in case of leakage or spill incident.

Regarding condition (ii), a test method for measuring the delta $E^*$ is indicated at the end of the description. Such absorbent article (7) creates a large enough delta E* to ensure that the indicia (30) can be distinguished enough from the sensor (6), namely to ensure that the indicia (30) are as visible as possible for sizing communication and ensuring the caregiver to quickly know which absorbent article (7) to apply whilst having the sensor (6) printing least visible or as subtle as possible, meaning subtle enough that the care giver can see the exudate detection technology in the absorbent article (7) but still to know which absorbent article (7), e.g. size or absorbency, to apply. Indeed, the exudate detection tracks, *i.e.* the sensor (6) and namely the connection tracks (5), occupy a significant visual space, meaning that the connection tracks take up a significant area of the backsheet (16), therefore important information such as size, absorbency, etc. can get lost and a care-giver cannot know which absorbent article (7) to apply if he/she cannot easily find this information. Hence having a large enough delta E* is preferable for a care-giver to be able to identify this information and know which absorbent article (7) to apply.

The indicia (30) can be arranged within the central portion (34), for example between the central portion of two connection tracks (5), or within the electrode portion (32) whether when printed on the same surface (garment-facing surface of the backsheet (16)) or whether when printed on the two different surfaces (body-facing and garment-facing surfaces of the backsheet (16)) and projected on one surface. According to an embodiment, the indicia (30) are partially covered by the insulating strip. According to another embodiment, none of the indicia (30) are covered by the insulating strip. According to another embodiment, some of the indicia (30) are covered by the insulating strip and other indicia (30) are not covered by the insulating strip. The indicia (30) that are covered by an insulating strip do not require to be printed in electric conductive material, as they are isolated from the connection tracks (5) and will not be in contact with exudates. In other words, in an embodiment of the present disclosure, some indicia (30) can comprise electrically conductive material and be arranged in the electrodes (32) portion to participate to exudate detection, whilst some indicia (30) do not comprise electrically conductive material but still have a first colour with the sensor (6) having at least a second colour and with a sufficient large enough delta $E^*$.

The present disclosure is not limited to this embodiment, the indicia (30) can be printed on both the body-facing and garment-facing surfaces of the backsheet (16) with non-electrically conductive material with a first colour to ensure a sufficient large enough delta E* with the colour of the sensor (6). The indicia (30) can also be printed on both the body-facing and garment-facing surfaces of the backsheet (16) with electrically conductive material. The indicia (30) can be printed on the garment-facing surface of the backsheet (16) with non-electrically conductive material with a first colour to ensure a sufficient large enough delta E* with the colour of the sensor (6) and on the body-facing surface of the backsheet (16) with electrically conductive material. The indicia (30) can be printed on the garment-facing surface of the backsheet (16) with non-electrically conductive material with a first colour to ensure a sufficient large enough delta $E^*$ with the colour of the sensor (6) and on the body-facing surface of the backsheet (16) with electrically conductive material and non-electrically conductive material with a first colour to ensure a sufficient large enough delta $E^*$ with the colour of the sensor (6).

Such indicia (30) enable to effectively communicate on the technology present on the absorbent article (7) whilst eventually helping in exudates detection. For example the indicia (30) may be an indication of one or more absorbent article properties, the one or more absorbent article properties preferably being selected from the group consisting of size, absorbency, brand, product type, and combinations thereof, more preferably wherein product type is selected from the group consisting of diapers, pants, pads, and combinations thereof.

According to an embodiment, the sensor (6) is positioned between backsheet (16) and absorbent core (15), preferably printed onto the body-facing surface of the backsheet (16).

According to an embodiment, the delta $E^*$ is from about 3.5 to about 30, preferably from about 4 to about 25, even more preferably from about 4.5 to about 20, most preferably from about 5 to about 15, as measured according to the test method herein. As explained hereabove, such values enable to communicate more effectively on the technology present in the absorbent article and enable the care-giver to isolate the information, e.g. the absorbent article (7) properties, from the connection tracks (5). For example the indicia (30) can give an indication of one or more absorbent article properties, the one or more absorbent article properties preferably being selected from the group consisting of size, absorbency, brand, product type, and combinations thereof, more preferably wherein product type is selected from the group consisting of diapers, pants, pads, and combinations thereof.

According to an embodiment, the indicia (30) is selected from the group consisting of graphics, letters, numbers, patterns, icons, images, and combinations thereof. Such indicia (30) enables to communicate more effectively information. For example, a letter can give an indication of size (e.g. S, M, L, XL) whereas absorbency can be illustrated with droplet(s) (e.g. 1 droplet - low absorbency, 3 droplets - high absorbency). According to an embodiment, the indicia (30) comprise different forms, e.g. letters and graphics, to communicate different information.

According to an embodiment, the indicia (30) comprises an electrically conductive material having one or more first conductive properties and the sensor (6) comprises an electrically conductive material having one or more second conductive properties, the first and second conductive properties being different. Preferably, the first conductive property comprises a first resistance and the second conductive property comprises a second resistance and wherein the first resistance is greater than the second resistance, preferably at least two times greater for example between 2 and 5 times greater. Such properties enables to give more information on the leakage. The system comprises means of detecting the difference between the resistance and know which indicia or track is connected hence under urine. In other words, having different resistance between the indicia (30) and the sensor (6) enables to lower threshold of detection of exudate by enhancing conductivity. For example, the indicia (30) and the sensor (6) can comprise the same electrically conductive material with each indicia (30) undergoing two or three printing process whereas the sensor (6) and the connection tracks (5) undergo only one printing process, hence the resistance of the indicia (30) will be two or three times greater than the sensor (6). In other words, according to an embodiment the indicia (30) are printed twice or thrice whereas the connection tracks (5) are only printed once. For example the indicia (30) and the sensor (6) can comprise different electrically conductive material with different properties. Inversely, the first resistance of the indicia (30) can be lesser than the second resistance of the sensor (6), for example the first resistance can be half or a third or a quarter of the second resistance. Such ratio will still induce a difference in signal and hence give more information on the exudate (wetness) event. Although, it is preferable that the first resistance be greater than the second resistance, printing the indicia (30) twice or thrice is less expensive than printing the connection tracks (5) twice or thrice.

According to an embodiment, one or more indicia (30) are positioned substantially between at least two electrodes (32) of the sensor 6, preferably wherein at least two of said indicia (30) are positioned between at least two same electrodes (32) of the sensor (6). As illustrated in FIG. 3, the electrodes (32) extend longitudinally and/or transversely, it is possible to arrange one or more indicia (30) or islands (31) between two electrodes. As illustrated in FIG. 3, according to an example, the sensor (6) comprise seven connection tracks (5). These seven connection tracks (5) each comprise a central portion (34) extending substantially longitudinally following either a linear or a curved direction and an electrode (32) portion extending outboard of the central portion (34). The electrodes (32) can comprise bifurcations hence a connection track (5) can comprise several electrodes (32). If we refer to these connection tracks as 5a to 5g, for example one or more indicia (30) can be arranged or positioned between the electrode (32a) of connection track 5a and the electrode (32b) of connection track 5b as illustrated in FIG. 3. One or more indicia (30) can be positioned between two electrodes (32) of one connection track.

According to an embodiment, the indicia (30) is viewable from a garment facing surface of the absorbent article (7). The care-giver can thus have access to the information, such as the absorbent article properties, more easily.

According to an embodiment, the indicia (30) is used for both exudate detection and indication of one or more absorbent article properties, the one or more absorbent article properties preferably being selected from the group consisting of size, absorbency, brand, product type, and combinations thereof, more preferably wherein product type is selected from the group consisting of diapers, pants, pads, and combinations thereof.

According to an embodiment, the first colour has an $L_1$ of at least about 35 and the second colour has an $L_2$ of at most about 40, as measured according to the test method herein.

According to an embodiment, $L_1$ is from about 40 to about 80, preferably from about 45 to about 75, even more preferably from about 50 to about 70, as measured according to the test method herein.

According to an embodiment, $L_2$ is from about 0 to about 35, preferably from about 5 to about 30, even more preferably from about 10 to about 25, even more preferably from about 15 to about 20, as measured according to the test method herein.

According to an embodiment, the first colour has an $a_1$ of from about -40 to about 40, preferably from about -30 to about 30, as measured according to the test method herein.

According to an embodiment, the first colour has a $b_1$ of from about -80 to about 20, preferably from about -75 to about 10, even more preferably from about -70 to about 0, as measured according to the test method herein.

Such hereabove values ($L_1, L_2, a_1, b_1$) ensures a large enough delta E* and thus enable to communicate more effectively on the technology present in the absorbent article and enable the care-giver to isolate the information, e.g. the absorbent article (7) properties, from the connection tracks (5).

According to an embodiment, a portion of the sensor (6), preferably one or more terminal tracks (33) of said sensor (6), are arranged to come into electrical contact with one or more conductive terminals of a reading device (3) preferably in the form of a clip-on device (3) as described herein.

The present disclosure also pertains to an array comprising plurality of absorbent articles (7) as described herein. Said array comprises a first absorbent article (7), a second absorbent article (7), and preferably a third absorbent article (7), and the indicia (30) of each said absorbent article is different and that the first, second, and third absorbent articles are different in at least one of size, absorbency, product type, and brand.

According to an embodiment, the sensor (6) for each said absorbent articles (7) is different in at least one of shape, size, area-coverage , number of electrodes.

There are person who use different incontinence products, namely who use the same kind of absorbent articles with different features namely different indicia, for example day-time and night-time incontinence pants, these pants having different absorbencies. It can also be interesting for example for the day-time absorbent articles (7) to have different indicia (30) than the night-time absorbent article (7). For example, the day-time absorbent articles (7) having a lower absorbency, can comprise indica (30) with electrically conductive material (cf. condition (i) in claim 1) on the body-facing side and with a large enough delta $E^*$ (cf. condition (ii) in claim 1) on the garment facing side of the backsheet (16) whereas the night-time absorbent articles (7) having a higher absorbency, can only have indicia (30) with a large enough delta $E^*$ (cf. condition (ii) in claim 1) on the garment facing side of the backsheet (16), detection being less crucial since the absorbent article (7) has a higher absorbency and hence less risk of leakage. Therefore, it might be opportune for these users to have absorbent articles (7) with different indicia (30) in the same array. The indicia (30) can vary based on their arrangement and/or material but also based on their shape and number, for example, the night-time absorbent articles (7) can comprise 3 droplets indicia (30) whereas the day-time absorbent articles (7) can comprise 1 droplet indicia (30), the night-time absorbent articles (7) can also comprise night-time themed indicia (30) (e.g. moon, ZZZ,...) and the day-time absorbent articles (7) can also comprise day-time themed indicia (30) (e.g. sun, ice cream,...). Of course the present disclosure is not limited to these day-time, night-time arrangement, the absorbent articles (7) in said array can vary based on size (S, M, L, XL) and/or absorbency and/or exudate detection capabilities by comprising different indicia (30). The absorbent articles (7) in said array can vary based on indicia (30) alone. As seen hereabove, the indicia (30) participate in exudate detection as well as in conveying information, having different indicia (30) can have an impact on these properties, e.g. having more indicia (30) can improve exudate detection.

The term "array" as used herein means a display of two or more packages, each comprising absorbent articles (7) with exudate detection sensors (6) and indicia (30). The various arrays described herein include at least one package comprising first (e.g. night-time, large size or blue-colored) absorbent articles (7) with sensor (6) and indicia (30), and at least one package comprising second (e.g. day-time, small size or green-colored) absorbent articles (7) with sensor (6) and indicia (30). The array may optionally include additional packages of one or both types of absorbent articles, for example, packages having different indicia or size or other features. The array may optionally include additional packages of other types of absorbent articles (7). One example of an array includes two or more packages having a same brand and/or sub-brand, and said packages are oriented in proximity to each other in a given area of a retail store. An array is marketed as a line-up of products normally having like packaging elements, e.g., packaging material type, film, paper, dominant color, design theme, etc. that convey to consumers that the different individual packages are part of a larger line-up. Arrays often have the same brand and same sub-brand. An array may have a same brand and different sub-brands. An array may include two packages that are distinctly different but together will be recognized as part of an array because of other shared elements. Arrays also often bear the same trademarks, such as trademarks of the brand, sub-brand, and/or features and/or benefits across the line-up. Arrays can be sold or distributed by a retail store or by an on-line source such as an e-commerce website or any platform enabling electronically buying or selling products on online services or over the Internet such as online shopping for retail sales direct to consumers via web sites or mobile apps. An array of products displayed and distributed on a common on-line source may be referred to as an "On-line Array."

THE SYSTEM

A system for monitoring at least one of exudate data and absorbent article data herein typically comprises: a sensor (6) comprising connection tracks (5) and indicia (30), said sensor (6) being associated with an absorbent article (7), which is arranged to generate an output signal representative of at least one of the location (and/or amount) of an exudate within said absorbent article (7), and one or more primary article characteristics of said absorbent article (7); and a data processing unit as described herein, said data processing unit being adapted to process said output signal generated by the sensor (6) and eventually the indicia (30).

The data processing unit for monitoring at least one of exudate data and absorbent article data is generally adapted to perform the method as described herein. The data processing unit may be comprised by the clip-on device (3), the computing device (2) and/or further computing device. Indeed activities of the data processing unit may be shared by any of the clip-on device (3), the computing device (2) and/or further computing device to limit power consumption related to the computing tasks executed, but may be equally carried out within only one or more of the clip-on device (3), the computing device (2) and further computing device such as by a processor and/or logic thereof.

The system may include an encoder and a decoder. One or more elements of the system may be integrated into a single computing device and/or distributed across multiple computing devices as described herein. For example, the encoder may be included in a first computing device, while the decoder may be included in a second computing device, the first computing device may be the clip-on device (3) and the second computing device may be the computing device (2) or the further computing device. The first computing device may be configured to transmit data to the second computing device in accordance with any suitable wireless and/or wired protocols.

The system may comprise the article herein and adapted to carry out a method for processing sensor signals representing a wetness event

THE METHOD

There can be several method for processing sensor signals representing a wetness event. Following is one example of method as described in EP3747415 A1, the present disclosure is not limited to this embodiment. For example publications EP3888607A1, EP3936100A1 or EP4014936A1 disclose other examples of method for processing sensor signals representing a wetness event and can be used with the present system.

One example of a method for processing sensor signals representing a wetness event in an absorbent article, the method comprising the steps of: providing an absorbent article comprising a sensor adapted to detect exudate (e.g. wetness) events therein and a first clip-on data processing module, i.e. clip-on device (3), in electrical communication with said sensor (6) and eventually indicia (30) e.g. during an exudate event; providing a database comprising a plurality of information factors, wherein said information factors comprise individualized thresholds for a plurality of absorbent article conditions, each said individualized thresholds being allocated with a corresponding warning condition; receiving from the sensor (6) and eventually indicia (30) signals representing a plurality of real-time indicators comprising wearing time; wetness events in an absorbent article; interval time between wetness events and/or duration of said wetness event within said absorbent article; processing the sensor (6) and eventually indicia (30) signals to determine an absorbent article condition; wherein processing the sensor (6) and eventually indicia (30) signals includes combining the information factors with the real-time indicators and, from the sensor (6) and eventually indicia (30) signals representative of each individual wetness event: (i) assigning a position of said wetness event within an x, y, (and optionally z) coordinates of the absorbent article; (ii) grouping wetness event indicators, such as amount of exudate within said wetness event, according to a common position according to step (i); (iii) normalizing the values of the real-time indicators, from the sensor (6) and eventually indicia (30) signals; (iv) allocating weightings to the normalised values to generate weighted values for that wetness event; and wherein the weighted values and the grouped wetness event indicators are combined to generate a combined event indicator that is compared with the information factors and when the combined event indicator is equal to one or more of the information factors a signal is generated by the clip-on data processing module according to the one or more corresponding warning conditions, e.g. an alarm sound such as a beeping noise.

Measurement of delta $E^*$ ($\Delta E^*$)

Delta $E^*$ or Delta E, is the difference between two colours designated as two points in the Lab colour space, with values assigned to each of the L, a, and b attributes of two colours. Lab colour space is comprised of three axes: $L$ represents darkness to lightness (achromatic), with values ranging from 0 to 100; a represents greenness to redness with values of -128 to +127 (chromatic); and $b$ represents blueness to yellowness also with values from -128 to +127 (chromatic). Following is one method for measurement of such colours difference:

First colour versus second colour difference analysis

The colour difference measurement is based on the CIE L* a* b* colour system (CIELAB). A flat bed scanner capable of scanning a minimum of 24-bit colour at 1200 dpi and has manual control of colour management (a suitable scanner is an Epson Perfection V750 Pro from Epson America Inc., Long Beach CA) may be used to acquire images. The scanner is calibrated against a colour reflection target compliant to ANSI method IT8.7/2-1993 using colour management software (a suitable package is MonacoEZColor available from X-Rite Grand Rapids, MI) to construct a scanner profile. The resulting calibrated scanner profile is opened within an imaging program that supports sampling in CIE L* a* b* (a suitable program is Photoshop S4 available from Adobe Systems Inc., San Jose, CA) to measure first and second colours of indicia and sensor respectively.

Turn on the scanner for 30 minutes prior to calibration. Place the IT8 target face down onto the scanner glass and close the scanner lid. Open the MonacoEZColor software and select acquire image using the Twain software included with the scanner. Within the Twain software deselect the unsharp mask setting and any automatic colour correction or colour management options that may be included in the software. If the automatic colour management cannot be disabled, the scanner is not appropriate for this application. Acquire a preview scan at 200 dpi and 24-bit colour. Ensure that the scanned image is straight and first outer surface facing side-up. Crop the image to the edge of the target, excluding all white space around the target, and acquire the final image. The MonacoEZColor software uses this image to compare with included reference files to create and export a calibrated colour profile compatible with Photoshop. After the profile is created the scan resolution (dpi) can be changed, but all other settings should be kept constant while imaging samples.

Identify an area of the backsheet that contains the indicia and sensor areas of interest (an area with at least a portion of one island of indicia and at least a portion of sensor is preferably captured in such area). Remove a piece of the backsheet corresponding to the area to be tested. For convenience of handling, the sample size may be a 75 mm by 75 mm piece, however, as will be appreciated by the person skilled in the art, smaller samples sizes can be used when suitable. If the backsheet needs to be removed from a product, such as an absorbent article, it may be necessary to use a cryogenic freeze spray (e.g. CytoFreeze, Control Company, TX) to remove the specimen from the product. Precondition samples at about 23°C $\pm$ 2 C° and about 50% $\pm$ 2% relative humidity for 2 hours prior to testing.

Open the scanner lid and place the specimen onto the scanner glass with the first outer surface facing the glass. Cover the specimen with the white background (in this test method white is defined as having L* > 94, -2 < a* < 2, and -2 < b* < 2) and close the lid. Acquire and import a scan of the specimen into Photoshop at 600 dpi and 24-bit colour. Assign the calibrated scanner profile to the image and change the mode to Lab Colour ("Lab Color" in Photoshop corresponds to the CIE L* a* b* standard). Select the "eyedropper" colour selection tool. Set the sampling size of the tool to include as many pixels as possible within an area of the indicia without including pixels from adjacent or nearby areas(s) of sensor. Using the eyedropper tool measure and record L* a* b* values in at least 10 different (may be randomly selected) indicia areas in the backsheet image. Average the 10 individual L* a* b* values and record as L1, a1, and b1 respectively. Repeat the measure in like fashion for 10 different sensor areas in the backsheet image, and record the averaged values as L2, a2 and b2. Calculate and report the colour difference (delta E*) between the bonded and unbonded areas using the following equation:

$$\text{delta } E^* = \sqrt{(L_2^* - L_1^*)^2 + (a_2^* - a_1^*)^2 + (b_2^* - b_1^*)^2}$$

and report to the nearest 0.01 units. A total of three substantially identical backsheets are preferably measured for each sample set. Average the three delta E* values and report to the nearest 0.1 unit.

Other colour analyses that may be useful are made using the calculations of delta Chroma (delta C*) and delta

Hue (delta H*) :

$$\text{Delta } C^* = \text{square-root}(a^*_1{}^2 + b^*_1{}^2) - \text{square-root}(a^*_2{}^2 + b^*_2{}^2)^2$$

$$\text{Delta } H^* = \text{square-root}[(a^*_2 - a^*_1)^2 + (b^*_2 - b^*_1)^2 - (\text{Delta}C^*)^2]$$

**Claims**

1.  An absorbent article (7) comprising:

    a. a liquid permeable topsheet (14)
    b. a substantially liquid impermeable backsheet (16)
    c. an absorbent core (15) positioned between said topsheet (14) and backsheet (16)
    d. a sensor (6) for exudate detection, said sensor comprising an electrically conductive material;

    **characterised in that** said absorbent article (7) further comprises indicia (30) positioned on at least one of a body-facing surface and garment-facing surface of the backsheet (16), and **in that** at least one of the following conditions is satisfied:

    (iii) said indicia (30) comprises an electrically conductive material and is arranged such that a plurality of islands (31) of said conductive material are formed on said backsheet (16); and
    (iv) said indicia (30) has at least a first colour and the sensor (6) has at least a second colour and wherein a delta $E^*$ between the first colour and second colour is at least about 3, as measured according to the test method herein.

2.  An absorbent article (7) according to claim 1, wherein the delta $E^*$ is from about 3.5 to about 30, preferably from about 4 to about 25, even more preferably from about 4.5 to about 20, most preferably from about 5 to about 15, as measured according to the test method herein.

3.  An absorbent article (7) according to any of the preceding claims, wherein the indicia (30) is selected from the group consisting of graphics, letters, numbers, patterns, icons, images, and combinations thereof.

4.  An absorbent article (7) according to any of the preceding claims, wherein the indicia (30) comprises an electrically conductive material having one or more first conductive properties and the sensor (6) comprises an electrically conductive material having one or more second conductive properties, and wherein the first and second conductive properties are different; preferably wherein the first conductive property comprises a first resistance and the second conductive property comprises a second resistance and wherein the first resistance is greater than the second resistance, preferably at least two times greater, more preferably between 2 and 5 times greater.

5.  An absorbent article (7) according to any of the preceding claims, wherein the indicia (30) are positioned substantially between at least two electrodes (32) of the sensor (6), preferably wherein at least two of said indicia (30) are positioned between at least two same electrodes (32) of the sensor (6).

6.  An absorbent article (7) according to any of the preceding claims, wherein the indicia (30) is viewable from a garment facing surface of the absorbent article (7).

7.  An absorbent article (7) according to any of the preceding claims, wherein the indicia (30) is used for both exudate detection and indication of one or more absorbent article properties, the one or more absorbent article properties preferably being selected from the group consisting of size, absorbency, brand, product type, and combinations thereof, more preferably wherein product type is selected from the group consisting of diapers, pants, pads, and combinations thereof.

8.  An absorbent article (7) according to any of the preceding claims, wherein the first colour has an $L_1$ of at least about 35 and the second colour has an $L_2$ of at most about 40, as measured according to the test method herein.

9. An absorbent article (7) according to claim 8, wherein $L_1$ is from about 40 to about 80, preferably from about 45 to about 75, even more preferably from about 50 to about 70, as measured according to the test method herein.

10. An absorbent article (7) according to claim 8, wherein $L_2$ is from about 0 to about 35, preferably from about 5 to about 30, even more preferably from about 10 to about 25, even more preferably from about 15 to about 20, as measured according to the test method herein.

11. An absorbent article (7) according to any of the preceding claims, wherein the first colour has an $a_1$ of from about -40 to about 40, preferably from about -30 to about 30, as measured according to the test method herein.

12. An absorbent article (7) according to any of the preceding claims, wherein the first colour has a $b_1$ of from about -80 to about 20, preferably from about -75 to about 10, even more preferably from about -70 to about 0, as measured according to the test method herein.

13. An absorbent article (7) according to any of the preceding claims, wherein a portion of the sensor (6), preferably one or more terminal tracks (33) of said sensor (6), are arranged to come into electrical contact with one or more conductive terminals of a reading device (3) preferably in the form of a clip-on device (3).

14. An array comprising plurality of absorbent articles (7) according to any of the preceding claims, wherein said array comprises a first absorbent article (7), a second absorbent article (7), and preferably a third absorbent article (7), and wherein the indicia (30) of each said absorbent article is different and that the first, second, and third absorbent articles are different in at least one of size, absorbency, product type, and brand.

15. An array according to claim 14, wherein the sensor (6) for each said absorbent articles (7) is different in at least one of shape, size, area-coverage , number of electrodes.

**FIG. 1**

FIG. 2

FIG. 3

FIG. 4

Europäisches
Patentamt

European
Patent Office

Office européen
des brevets

# EUROPEAN SEARCH REPORT

**Application Number**

EP 23 20 9859

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/129630 A1 (WEBER AINAS [DE] ET AL) 27 April 2023 (2023-04-27) | 1-4,6-15 | INV. A61F13/42 A61F13/514 |
| A | * paragraphs [0009], [0052] - [0055], [0188], [0189]; figure 1 * | 5 | |
| A | US 2019/167489 A1 (HELLMOLD JENS [DE] ET AL) 6 June 2019 (2019-06-06) * paragraphs [0016], [0018], [0023], [0098] * | 1-15 | |
| A | EP 4 014 936 A1 (ONTEX BV [BE]; ONTEX GROUP NV [BE]) 22 June 2022 (2022-06-22) * paragraphs [0018] - [0022], [0041], [0054] * | 1-15 | |

**TECHNICAL FIELDS SEARCHED (IPC)**

A61F

The present search report has been drawn up for all claims

| Place of search | Date of completion of the search | Examiner |
|---|---|---|
| Munich | 9 April 2024 | Beins, Ulrika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................

& : member of the same patent family, corresponding document

EPO FORM 1503 03.82 (P04C01)

# EP 4 497 427 A1

## ANNEX TO THE EUROPEAN SEARCH REPORT
## ON EUROPEAN PATENT APPLICATION NO.

EP 23 20 9859

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2024

| Patent document cited in search report | | | Publication date | Patent family member(s) | | | Publication date |
|---|---|---|---|---|---|---|---|
| US 2023129630 | A1 | | 27-04-2023 | EP | 3888608 | A1 | 06-10-2021 |
| | | | | EP | 4125757 | A1 | 08-02-2023 |
| | | | | ES | 2954670 | T3 | 23-11-2023 |
| | | | | US | 2023129630 | A1 | 27-04-2023 |
| | | | | WO | 2021198460 | A1 | 07-10-2021 |
| US 2019167489 | A1 | | 06-06-2019 | AU | 2018278900 | A1 | 17-01-2019 |
| | | | | AU | 2018278912 | A1 | 17-01-2019 |
| | | | | EP | 3415130 | A1 | 19-12-2018 |
| | | | | EP | 3451988 | A1 | 13-03-2019 |
| | | | | EP | 3461257 | A2 | 03-04-2019 |
| | | | | EP | 3638178 | A2 | 22-04-2020 |
| | | | | EP | 3638179 | A1 | 22-04-2020 |
| | | | | EP | 3638180 | A1 | 22-04-2020 |
| | | | | ES | 2834924 | T3 | 21-06-2021 |
| | | | | ES | 2889586 | T3 | 12-01-2022 |
| | | | | US | 2019167489 | A1 | 06-06-2019 |
| | | | | US | 2019167490 | A1 | 06-06-2019 |
| | | | | US | 2020196933 | A1 | 25-06-2020 |
| | | | | US | 2022249298 | A1 | 11-08-2022 |
| | | | | WO | 2018228822 | A2 | 20-12-2018 |
| | | | | WO | 2018229118 | A2 | 20-12-2018 |
| | | | | WO | 2018229121 | A1 | 20-12-2018 |
| | | | | WO | 2018229280 | A1 | 20-12-2018 |
| EP 4014936 | A1 | | 22-06-2022 | EP | 4014936 | A1 | 22-06-2022 |
| | | | | WO | 2022129297 | A1 | 23-06-2022 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A
- WO 2012052172 A
- EP 2496197 B1
- EP 2739254 B1
- EP 2582341 B1
- EP 3415130 A
- WO 2018228822 A
- WO 2018229017 A
- EP 3461257 A
- EP 3451988 A
- EP 3760104 A
- EP 3747415 A1
- EP 3888607 A1
- EP 3936100 A1
- EP 4014936 A1